# EUROPÄISCHE PATENTANMELDUNG

(11) **EP 1 438 987 A1**
(43) Veröffentlichungstag der Anmeldung: **21.07.2004**
(21) Anmeldenummer: 03000953.4
(22) Anmeldetag: 16.01.2003
(51) Int. Cl.: A61N 2/00, A61N 1/32, A61N 1/08

(54) **Therapiegerät**

(71) Anmelder: ProCare Produktions- & Handels GmbH, 6911 Lochau b. Bregenz (AT)
(72) Erfinder: Schaknat, Carsten, 92358 Seubersdorf-Ittelhofen (DE); Imlauer, Georg, 6911 Lochau (AT)
(74) Vertreter: Schurack, Eduard F.

(57) **Zusammenfassung**

Die Erfindung betrifft ein Therapiegerät (1) dadurch gekennzeichnet, dass dieses einen Generator (17) zum Erzeugen eines Mircrostroms zur Behandlung eines Patienten und zusätzlich einen Generator (18) zur Erzeugung eines Magnetfelds zur Behandlung eines Patienten aufweist.

## Beschreibung

Die vorliegende Erfindung betrifft ein Therapiegerät, insbesondere ein Therapiegerät für eine Kombinationsbehandlung mittels Strom.

Zur Behandlung verschiedener Erkrankungen und/oder Beschwerden von Patienten hat sich herausgestellt, dass durch Nachahmung des bioelektrischen Systems der natürliche Ausgleich gestörter Zellsysteme erreicht werden kann und damit Heilungsprozesse eingeleitet und unterstützt werden können. Weiterhin ist erkannt worden, dass durch Erzeugung eines elektromagnetischen Biofelds bioenergetisch lebenswichtige Signale für den Menschen nachgebildet werden können, die einer Behandlung und Heilung verschiedener Indikationen dienen können.

Zu diesem Zweck sind Microstrom-Therapiegeräte entwickelt worden, bei denen ein Strom, dessen Intensität im Microampèrebereich und dessen Frequenz im Bereich von 0,01 Hz bis 1000 Hz liegt, dem Bereich, in dem Nervenfasern erregbar sind, beispielsweise über Pads, die auf die Haut zeitweise aufgeklebt werden können, in den menschlichen Körper eingebracht werden.

Diese Geräte werden in der Regel von Therapeuten bedient, die über die gezielte Einstellung von der Frequenz, der Intensität und weiterer Variablen eine Behandlung des Patienten durchführen können. Weiterhin sind Magnetfeld-Therapiegeräte bekannt, bei denen die in der Natur vorkommenden harmonischen und für den Menschen bioenergetisch lebenswichtigen Schwingungen erzeugt werden können. Diese Magnetfeld-Therapiegeräte können in Form eines Handgeräts hergestellt werden, das von den Patienten unabhängig von einem Therapeuten verwendet werden kann.

Der Nachteil der bekannten Geräte liegt darin, dass für eine Behandlung mittels Microstrom und eine Behandlung mittels Magnetfeld getrennte Geräte verwendet werden müssen. Insbesondere für den Patienten ist eine Kombination der beiden Behandlungen nicht möglich, da in der Regel die Einstellungen eines Microstromgeräts von einem Therapeuten oder Arzt durchgeführt werden müssen.

Aufgabe der vorliegenden Erfindung ist es ein Therapiegerät zu schaffen, das ein gleichzeitiges Therapieren eines Patienten über unterschiedliche Therapiemittel erlaubt und das vorzugsweise von den Patienten selber bedient werden kann.

Diese Aufgabe wird erfindungsgemäß durch ein Therapiegerät gelöst, das sich dadurch auszeichnet, dass dieses einen Generator zum Erzeugen eines Microstroms zur Behandlung eines Patienten und zusätzlich einen Generator zum Erzeugen eines Magnetfelds zur Behandlung eines Patienten aufweist.

Der Generator kann eine Strom- und/oder Spannungquelle umfassen, die die gewünschten Signale bereitstellen/bereitstellt.

Der Vorteil dieses Kombinationsgeräts liegt darin, dass eine verbesserte Behandlung, wie beispielsweise die Heilung oder die Schmerzbehandlung, durch die beiden Therapien gleichzeitig durchgeführt werden kann. Wird das Kombinationsgerät beispielsweise zur Heilung eingesetzt, so wird durch den Microstrom ein Strom in das Gewebe der Haut eingebracht, der eine Stromstärke aufweist, die in biologischen Stromkreisläufen, insbesondere im Gewebe des Menschen gemessen werden kann. Hierdurch wird eine Heilung beschleunigt. Darüber hinaus können durch das erzeugte Magnetfeld harmonische Schwingungen in den menschlichen Körper eingebracht werden, die die Selbstheilung fördern. Die harmonischen Schwingungen werden vom Organismus als Steuerimpulse für eine einwandfreie Funktion ihrer inneren Regelkreise benötigt. Wie sich in der Praxis gezeigt hat, lassen sich durch eine Kombination der beiden Behandlungsmethoden Ergebnisse erzielen, die weit über der Summe der Ergebnisse der beiden Einzelbehandlungsmethoden liegen, so daß von einem synergetischen Effekt ausgegangen werden muß, der in dieser Form bisher nicht bekannt war.

Gemäß einer Ausführungsform weist das erfindungsgemäße Therapiegerät ein Steuergerät auf, das wahlweise zum Ansteuern des Generators zum Erzeugen eines Microstroms und/oder des Generators zum Erzeugen eines Magnetfelds verwendet werden kann. Durch das Vorsehen eines einzigen Steuergeräts, das sowohl für den Microstromgenerator, als auch für den Magnetfeldgenerator verwendet werden kann, wird die Herstellung und Bedienung des Geräts weiterhin erleichtert. Für den Therapeuten ist es daher einfach, die Microstrom-Therapie mit der Magnetfeld-Therapie zu kombinieren und gleichzeitig zu verabreichen.

Das Therapiegerät kann erfindungsgemäß eine Sperreinrichtung aufweisen, die eine gleichzeitige Aktivierung des Magnetfeld-Generators und des Microstrom-Generators verhindert. Diese Sperreinrichtung kann in Form einer Software oder in Form einer elektrischen Schaltung vorliegen. Durch eine solche Sperreinrichtung kann der versehentliche gleichzeitige Einsatz von Magnetfeld und Microstrom vermieden werden. Insbesondere für Patienten, die das Therapiegerät in der Heimanwendung einsetzen, kann es sinnvoll sein, im Hinblick auf den noch nicht vollständig erforschten Wirkungsmechanismus einer Kombination von Microstrom- und Magnetfeldtherapie, eine derartige Kombinationsbehandlung zu verhindern.

Vorzugsweise kann die Sperreinrichtung deaktiviert werden. Die Deaktivierung kann beispielsweise über einen Code erfolgen. So ist es beispielsweise für einen Therapeuten möglich, mit dem Gerät eine Kombinationsbehandlung zu verabreichen.

In einer weiteren Ausführungsform weist das Therapiegerät einen Speicher auf, in dem Indikationen jeweils zumindest einem Satz an Anweisungen für das Steuergerät zugeordnet sind.

Durch diese Ausgestaltung des Therapiegeräts wird es möglich, das Gerät für die Bedienung durch den Patienten freizugeben. Dieser kann durch Auswahl einer Indikation automatisch die für diese Indikation erforderlichen Einstellungen an dem Steuergerät aufrufen, ohne eine therapeutische Vorkenntnis besitzen zu müssen. Insbesondere kann durch die Zuordnung von Anweisungen an das Steuergerät zu Indikationen vermieden werden, dass ein Patient versehentlich eine gleichzeitige Microstrom- und Microtherapie bei Behandlung einleitet. Eine solche Kombination kann bei dem erfindungsgemäßen Therapiegerät nur durch den Therapeuten veranlasst werden, sofern bestimmte Indikationen bzw. Indikationsbereiche eine kombinierte Behandlung erfordern.

Um dem Therapeuten bzw. Arzt die Möglichkeit zu geben, die Zuordnung von Anweisungen für das Steuergerät zu bestimmten Indikationen vorzunehmen, kann das Therapiegerät eine Eingabevorrichtung umfassen.

Diese Eingabevorrichtung kann Teil des Therapiegeräts sein oder lösbar mit diesem verbunden werden. Bevorzugt wird die Eingabevorrichtung ein separates Gerät darstellen, das mit der Schnittstelle zur Eingabe von Daten rates Gerät darstellen, das mit der Schnittstelle zur Eingabe von Daten in den Speicher des Therapiegeräts verbunden werden kann. Ein solches Eingabegerät kann beispielsweise von einem Therapeuten bedient werden, der die einzustellenden Anweisungen an das Steuergerät den Indikationen zuweisen kann oder eine gegebenenfalls vorhandene Sperreinrichtung, zum Beispiel für bestimmte Indikationen, deaktivieren kann. Alternativ kann eine Liste von Indikationen mit diesen zugewiesenen Anweisungssätzen bei der Herstellung des Therapiegeräts in diesem vorprogrammiert werden. Der Vorteil einer Eingabevorrichtung, über die die Zuordnung der Anweisungssätze zu den Indikationen erfolgen kann, liegt darin, dass ein Therapeut diese Zuweisungen personenspezifisch durchführen kann und beispielsweise eine Kombinationsbehandlung von Magnetfeldtherapie und Microstromtherapie vorprogrammieren kann, der sich dann der Patient beim Abrufen dieser Vorprogrammierung unterziehen kann. Je nach Krankheitsbild, Alter und dergleichen können zur Behandlung einer gewissen Indikation unterschiedliche Variablen der einzelnen Therapien oder einer Kombinationstherapie notwendig sein. Über die Eingabevorrichtung kann zusätzlich ein Katalog von Indikationen in den Speicher des Therapiegeräts eingespeist werden. Alternativ kann dieser Katalog an Indikationen bereits bei der Herstellung des Therapiegeräts vorprogrammiert werden.

Um einem Missbrauch vorzubeugen, kann das Eingabegerät wahlweise nur von Therapeuten vertrieben werden, oder es kann eine Zugriffssicherung auf den Speicher vorgesehen sein.

Diese Zugriffssicherung kann in Form einer Verschlüsselung der gespeicherten Daten ausgeführt sein. Für den Zugriff dieser verschlüsselten Daten kann ein Code benutzt werden, wodurch ein Zugriff durch Unberechtigte vermieden werden kann. Insbesondere kann durch diese Zugriffssicherung verhindert werden, dass ein Patient die gespeicherten Daten ändert und dadurch die Gefahr der Verletzung bzw. das Ausbleiben des Therapieerfolgs hervorruft. Ein Code, der die Zugriffssicherung darstellen kann, sollte vorzugsweise ausschließlich durch einen geschulten Therapeuten eingegeben werden können.

Der Bereich, in dem die Intensität des Microstroms liegt, der von dem Therapiegerät erzeugt wird, ist vorzugsweise 5 bis 800 µA, besonders bevorzugt 10 bis 500 µA.

Die Erfindung wird im Folgenden anhand der beiliegenden Figuren genauer beschrieben, wobei:
- Figur 1:: eine schematische Blockansicht eines erfindungsgemäßen Therapiegeräts; und
- Figur 2:: eine schematische Darstellung einer Auswahleinheit zeigt.

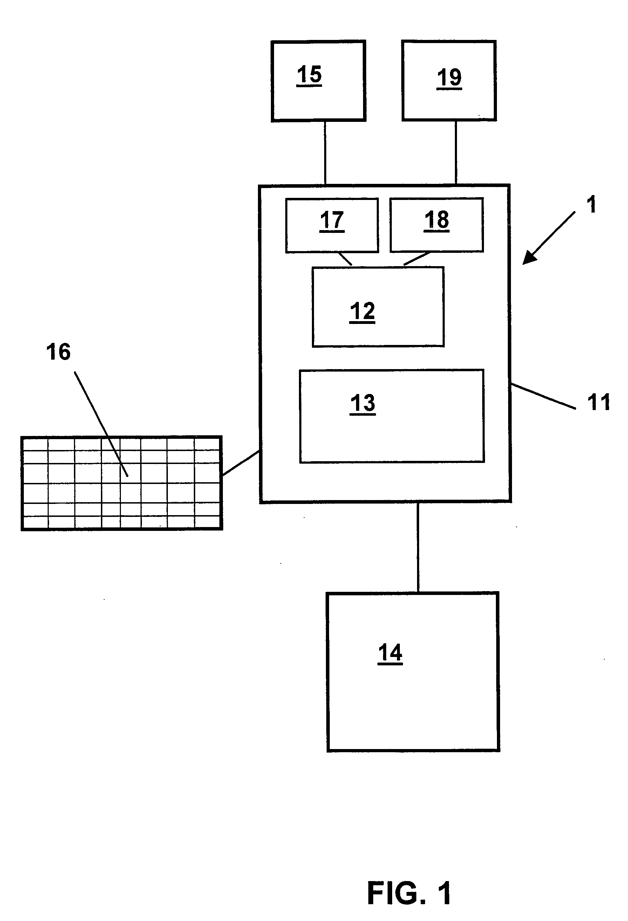

Das Therapiegerät 1 umfasst in der dargestellten Ausführungsform in einem Gehäuse 11 einen Generator 17 zum Erzeugen eines Microstroms, sowie einen Generator 18 zum Erzeugen eines Magnetfelds. Die beiden Generatoren sind in der dargestellten Ausführungsform über ein Steuergerät 12 mit einem Speicher 13 sowie einer Auswahlvorrichtung 14 verbunden. Die Generatoren 17, 18 sind mit Ausgabevorrichtungen 15, 19 verbunden, über die das Magnetfeld bzw. der Microstrom ausgegeben werden kann. Weiterhin ist die Auswahleinheit 14 ebenfalls außerhalb des Gehäuses 11, aber mit diesem verbunden, angedeutet. Die Eingabevorrichtung 16 ist in Figur 1 mit dem Gehäuse 11 und insbesondere mit dem in dem Gehäuse 11 befindlichen Speicher 13 verbunden.

Die Funktionsweise eines erfindungsgemäßen Therapiegeräts wird nunmehr unter Bezugnahme auf Figur 1 beschrieben.

Über die Eingabevorrichtung 16 können in den Speicher 13 Daten und Informationen eingegeben bzw. darin abgespeicherte Daten und Informationen geändert werden. Vorzugsweise wird von einem Arzt oder Therapeuten über die Eingabevorrichtung 16 ein Katalog von Indikationen in den Speicher 13 abgelegt. Die Indikationen, die in dem Katalog enthalten sind, werden weiterhin, vorzugsweise ebenfalls über die Eingabevorrichtung 16, mit weiteren Daten, die Anweisungen an das Steuergerät darstellen, in dem Speicher 13 in Bezug gebracht. Die Anweisungen an das Steuergerät 12 können insbesondere die Anweisung enthalten den Microstrom-Generator 17 und gleichzeitig den Magnetfeld-Generator 18 mit bestimmten Parametern, wie Frequenz und Intensität, zu aktivieren. Wird ein so vorprogrammiertes Therapiegerät 1 von einem Patienten bedient, so kann sich dieser mittels der Auswahleinheit 14 zunächst die in dem Speicher 13 abgelegten Indikationen anzeigen lassen. Aus diesen kann der Patient die für seine Beschwerden zutreffende Indikation auswählen. Von der Auswahleinheit wird diese gewählte Indikation an das Steuergerät 12 geleitet, das daraufhin aus dem Speicher 13 die Anweisungen, die dieser Indikation zugeordnet sind, ausliest. Entsprechend den ausgelesenen Anweisungen stellt das Steuergerät 12 Variablen ein, die insbesondere Steueranweisungen an einen oder beide Generatoren 17, 18 und / oder die Ausgabevorrichtungen 15, 19 sein können. Das Steuergerät 1 kann als Stromquelle eine Batterie verwenden, oder an ein Stromnetz angeschlossen werden.

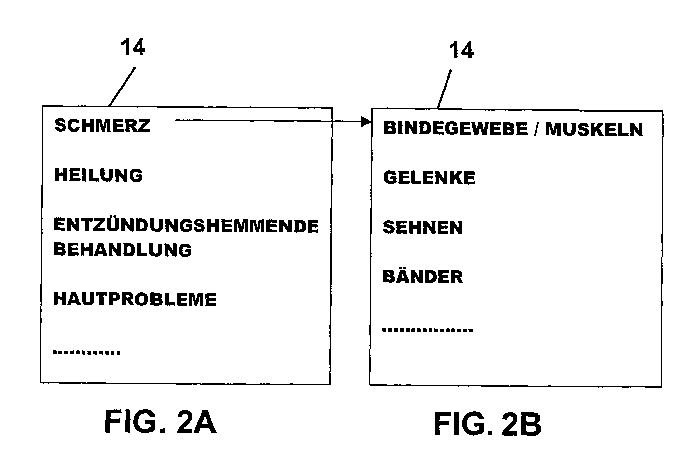

In Figur 2 ist eine mögliche Anzeige an der Auswahleinheit 14 dargestellt. Schaltet der Benutzer das Therapiegerät 1 an, so kann beispielsweise eine Auflistung von Indikationsbereichen, wie in Figur 2A angedeutet angezeigt werden. Indikationsbereiche können beispielsweise Schmerz, Heilung, entzündungshemmende Behandlung, Hauptprobleme, Relaxation, craniale Behandlung, Immunstimulation, antibakterielle Behandlung, Magen-Darm-Probleme, Nervenregeneration, Lymphdrainage, Ästhetikprogramm sein. Wählt der Patient beispielsweise den Indikationsbereich Schmerz aus, so wird an der Auswahleinheit 13 die Liste der Indikationen, die diesem Indikationsbereich zugehören, angezeigt. Eine solche Anzeige ist in Figur 2B beispielhaft dargestellt. Einzelindikationen können beispielsweise Bindegewebe/Muskeln, Gelenke, Sehnen oder Bänder sein. Wählt der Benutzer eine dieser Indikationen beispielsweise Sehnenschmerz aus, so wird über das Steuergerät 12 der dieser Indikation zugeordnete Satz an Anweisungen aus dem Speicher 13 ausgelesen. Der Satz der Anweisungen, der für die entsprechende Indikation in dem Speicher 13 vorprogrammiert ist, umfasst insbesondere die Frequenz, die Intensität, die Polarität, sowie die Übertragungswelle des zu verwendenden Stroms und bestimmt die Behandlungszeit. Der Satz an Anweisungen kann Anweisungen für die Erzeugung und Ausgabe eines Magnetfelds und / oder für die Erzeugung und Ausgabe eines Microstroms umfassen. Die Frequenz kann beispielsweise im Bereich von 0,01 bis 1000,00 Hz auf 1/100 genau vorprogrammiert werden. Die Intensität wird vorzugsweise im Bereich von 10 bis 500µA festgelegt. Bei der Polarität des Stromes kann monopolar positiv, monopolar negativ oder alternierend vorprogrammiert sein, wobei die Polarität bei der alternierenden Einstellung frequenzabhängig, beispielsweise ca. alle 3 Sekunden, wechselt. Die Übertragungswelle stellt vorzugsweise eine modulierbare Rechteckwelle dar. Die Behandlungszeit, die für die einzelnen Indikationen vorprogrammiert sein kann, kann im Bereich von 30 Minuten bis zu 8 Stunden liegen.

Die vorliegende Erfindung ist nicht auf die dargestellten Ausführungsformen beschränkt. Die Ausgabevorrichtung des Therapiegeräts kann beispielsweise Pads darstellen, die an der Haut des Patienten befestigt werden können, zusätzlich kann eine Ausgabevorrichtung in dem Gehäuse des Therapiegeräts aufgenommen sein und beispielsweise für die Ausgabe eines Magnetfelds dienen.

Auch die Auswahleinheit kann, anders als in den Figuren dargestellt, in dem Gehäuse aufgenommen sein und beispielsweise lediglich einen Bildschirm an diesem Gehäuse darstellen, über den eine Auswahl erfolgen kann. Die Eingabevorrichtung, über die Daten in das Therapiegerät, insbesondere in den Speicher des Therapiegeräts eingelesen bzw. geändert werden können, kann ein separates Gerät darstellen, das lediglich in das Therapiegerät eingesteckt wird, während die notwendigen Daten übertragen werden. Weiterhin ist es möglich, dass die Auswahleinheit unmittelbar mit der Speichereinheit zusammenwirkt, so dass vom Speicher die Sätze an Anweisungen ausgelesen werden, die der ausgewählten Indikation zugeordnet sind, ohne dass das Steuergerät zwischengeschaltet ist.

Als Sätze von Anweisungen können neben einzelnen Werten für beispielsweise die Intensität oder die Frequenz auch Bereiche vorprogrammiert sein. So kann beispielsweise nicht nur jede einzelne Frequenz im Bereich von 0,01 bis 1000 Hz individuell angesteuert werden, sondern es sind auch frequenzüberlagernde Therapien und Therapien mit Vektoreffekt möglich. Weiterhin ist es möglich, dass der Satz von Anweisungen bezüglich der Übertragungswelle Informationen enthält, so dass diese insbesondere im Bereich der ersten Sekunde der Therapie, insbesondere der Stimulation, individuell modulierbar ist. Diese individuelle Modulation kann beispielsweise durch den Therapeuten ebenfalls in dem Speicher personenspezifisch einprogrammiert sein.

Die Indikationsbereiche, die bei einer Ausführungsform der Erfindung zunächst über die Auswahleinheit ausgewählt werden können, können entweder einzelne Indikationen oder Indikationsbereiche zusammenfassen. So ist es beispielsweise möglich, als Indikationsbereiche medizinische Anwendungen, medikamentöse Anwendungen, Massage und dergleichen vorzuprogrammieren. Dem Indikationsbereich "medizinische Anwendung" kann dann beispielsweise der weitere Indikationsbereich "Schmerztherapie" zugeordnet sein, der sich in die einzelnen Indikationen Muskelschmerzen, Sehnenschmerzen etc. unterteilt. Erfindungsgemäß sind vorzugsweise den einzelnen Indikationen Sätze von Anweisungen für das Steuergerät zugeordnet. Es liegt aber auch im Rahmen der Erfindung einen Indikationsbereich einem Satz von Anweisungen zuzuordnen.

Mit dem erfindungsgemäßen Therapiegerät und dem erfindungsgemäßen System wird daher zum einen die Möglichkeit geschaffen eine Kombinationstherapie aus einer Magnetfeldtherapie und einer Microstromtherapie mittels eines Geräts ausführen zu können. Zum anderen kann die Möglichkeit für einen Patienten geschaffen werden, eine Therapie, wie beispielsweise eine Microstromtherapie und / oder eine Magnetfeldtherapie ohne ärztliche bzw. therapeutische Beobachtung durchführen zu können und ohne das Risiko in Kauf nehmen zu müssen, dass versehentlich eine Kombinationstherapie durchgeführt wird. Weiterhin bietet das erfindungsgemäße Therapiegerät und das erfindungsgemäße System einen hohen Grad an Sicherheit gegen falsche Behandlung, die gegebenenfalls zu Verletzungen, oder zumindest zu verschlechterten Behandlungsergebnissen führen kann. Besonders bevorzugt werden die in dem Speicher des Therapiegeräts abgelegten Daten personenspezifisch definiert. Dies kann insbesondere durch einen Therapeuten bzw. einen Arzt erfolgen. Gemäß der Erfindung kann ein Zugriff für andere Personen, beispielsweise mittels eines Codes, vermieden werden und insbesondere die Kombination von Magnetfeld- und Microstromtherapie ausschließlich durch befugte Personen durchgeführt werden.

## Patentansprüche

1. Therapiegerät (1) **dadurch gekennzeichnet, dass** dieses einen Generator (17) zum Erzeugen eines Mircrostroms zur Behandlung eines Patienten und zusätzlich einen Generator (18) zur Erzeugung eines Magnetfelds zur Behandlung eines Patienten aufweist.

2. Therapiegerät nach Anspruch 1, **dadurch gekennzeichnet, dass** dieses ein Steuergerät (12) aufweist, das wahlweise zum Ansteuern des Generators (17) zum Erzeugen eines Microstroms, und/oder des Generators (18) zum Erzeugen eines Magnetfelds verwendet werden kann.

3. Therapiegerät nach Anspruch 1 oder 2, **dadurch gekennzeichnet, dass** dieses eine Sperreinrichtung aufweist, die eine gleichzeitige Aktivierung des Magnetfeld-Generators (18) und des Microstrom-Generators (17) verhindert.

4. Therapiegerät nach Anspruch 3, **dadurch gekennzeichnet, dass** die Sperreinrichtung deaktiviert werden kann.

5. Therapiegerät nach einem der Ansprüche 1 bis 4, **dadurch gekennzeichnet, dass** dieses einen Speicher (13) aufweist, in dem Indikationen jeweils zumindest ein Satz an Anweisungen für das Steuergerät (12) zugeordnet sind.

6. Therapiegerät nach Anspruch 3, **dadurch gekennzeichnet, dass** die Zuordnung des mindestens eine Satzes an Anweisungen für das Steuergerät (12), zu der jeweiligen Indikation durch eine Eingabevorrichtung (16) erfolgt.

7. Therapiegerät nach Anspruch 1 bis 4, **dadurch gekennzeichnet, dass** in dem Therapiegerät (1) ein Mircostrom mit einer Intensität von 5 bis 800µA, vorzugsweise 10-500µA erzeugt werden kann.
